# EUROPEAN PATENT APPLICATION

(11) **EP 4 122 519 A1**
(43) Date of publication of application: **25.01.2023**
(21) Application number: 22183828.7
(22) Date of filing: 08.07.2022
(51) Int. Cl.: A61M 25/01, A61M 29/00

(54) **INTERMEDIATE CATHETER ACCESS ASSIST TO CEREBRAL ARTERIES**

(30) Priority: 09.07.2021 US 202163220183 P
(71) Applicant: Neuravi Limited, Galway H91 K5YD (IE)
(72) Inventor: VALE, David, Galway, H91 K5YD (IE); CASEY, Brendan, Galway, H91 K5YD (IE); CONLON, Richard, Galway, H91 K5YD (IE); KEATING, Karl, Galway, H91 K5YD (IE); MIRZA, Mahmood, Galway, H91 K5YD (IE); JOHNSON, Kirk, Galway, H91 K5YD (IE); POSADA, Jorge, Galway, H91 K5YD (IE); ECHARRI, Roberto, Galway, H91 K5YD (IE); RUIZ, Andres, Galway, H91 K5YD (IE); FONSECA, Dennis, Galway, H91 K5YD (IE)
(74) Representative: Carpmaels & Ransford LLP

(57) **Abstract**

A cerebral vessel access device (200) having a base tube (204) comprising an inner lumen (206), a shapeable element (208) disposed to one side of the base tube, and an outer jacket (210) covering the base tube and a portion of the shapeable element. The shapeable element can be configured to deflect from an axis line causing the base tube and outer jacket to deflect along with the shapeable element.

## Description

### Cross-Reference to Related Application

The present application claims the benefit of and priority to U.S. Provisional Patent Application No. 63/220,183 filed July 9, 2021. The entire contents of which are hereby incorporated by reference.

### Field of the Invention

This invention relates to devices and methods to assist access to the Internal Carotid Artery (ICA) and the middle cerebral artery.

### Background

In cases where patients are suffering from conditions such as acute ischemic stroke (AIS), acute obstructions may include clot, misplaced devices, migrated devices, large emboli, and the like. Thromboembolism occurs when part or all of a thrombus breaks away from the blood vessel wall. This clot (now called an embolus) is then carried in the direction of blood flow. An ischemic stroke may result if the clot lodges in the cerebral vasculature. There are a number of access challenges that make it difficult to deliver devices to these obstructions.

One challenge is in the tortuosity of the arteries approaching the brain. For example, it is not unusual at the distal end of the internal carotid artery that the device will have to navigate a vessel segment with a 180° bend, a 90° bend and a 360° bend in quick succession over a few centimeters of vessel. Another challenge is that the vasculature in the area in which the clot may be lodged is often fragile and delicate. For example, neurovascular vessels are more fragile than similarly sized vessels in other parts of the body and are in a soft tissue bed. Excessive tensile forces applied on these vessels could result in perforations and hemorrhage. What is needed is a device and method to assist in accessing these challenging locations.

### Summary

The present invention discloses examples to aid the operator in traversing the cerebral vessels. Examples of an access device includes a shapeable and tapered dilator which allows the access device to be directed through an intermediate catheter and maneuver through the tortious vasculature.

Other examples include a lock and rail catheter. In these examples the distal end of the rail catheter can engage the vessel wall and can anchor the distal end when tension is applied to the catheter. In use, the operator brings up base, aspiration, and rail catheters until, or before, the ophthalmic ostium or wherever the base and aspiration catheters get stuck on the vasculature. The operator then extends the rail catheter 302 to the clot and anchors the engaging element to the vessel wall. Once anchored, the operator can apply tension to the rail catheter causing both the catheter and the vascular to straighten. This straightening draws the rail catheter 302 away from the ophthalmic ostium, allowing operator to further deploy one or both of the base and aspiration catheters along the rail catheter and up to the clot.

To the accomplishment of the foregoing and related ends, certain illustrative aspects are described herein in connection with the following description and the appended drawings. These aspects are indicative, however, of but a few of the various ways in which the principles of the claimed subject matter may be employed and the claimed subject matter is intended to include all such aspects and their equivalents. Other advantages and novel features may become apparent from the following detailed description when considered in conjunction with the drawings.

### Brief Description of the Drawings

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1 shows a patient catheterized via femoral access with a clot located in a cerebral vessel using the arterial system for its delivery.
FIG. 2 shows certain anatomy of cerebral arteries above the aortic arch leading to the brain.
FIG. 3 illustrates an enlarged view of the internal carotid artery and ophthalmic ostium along with an access device of the present invention.
FIG. 4 is a partially sectioned side view of an example of an access aid of the present invention.
FIG. 5 is a partially dissembled side view of the example of the access aid of FIG. 4.
FIG. 6 is a partially transparent side view of the example of the access aid of FIG. 4.
FIG. 7 is another example of an access aid of the present invention.
FIGs. 8A, 8B and 8C illustrate an example of an engaging element of the present invention.
FIGs. 9A, 9B, 9C and 9D illustrate other examples of an engaging element of the present invention.
FIGs. 10A, 10B, 10C, 10D, 10E, and 10F illustrate further examples of an engaging element of the present invention.
FIGs. 11A and 11B illustrate slimmer profile examples of an engaging element/rail catheter of the present invention.
FIG 12 illustrates an example of an engaging element of the present invention.
FIG. 13 is a flow chart illustrating an example method of using a rail catheter.

### Detailed Description

Although example embodiments of the disclosed technology are explained in detail herein, it is to be understood that other embodiments are contemplated. Accordingly, it is not intended that the disclosed technology be limited in its scope to the details of construction and arrangement of components set forth in the following description or illustrated in the drawings. The disclosed technology is capable of other embodiments and of being practiced or carried out in various ways.

It must also be noted that, as used in the specification and the appended claims, the singular forms "a," "an" and "the" include plural referents unless the context clearly dictates otherwise. By "comprising" or "containing" or "including" it is meant that at least the named compound, element, particle, or method step is present in the composition or article or method, but does not exclude the presence of other compounds, materials, particles, method steps, even if the other such compounds, material, particles, method steps have the same function as what is named.

In describing example embodiments, terminology will be resorted to for the sake of clarity. It is intended that each term contemplates its broadest meaning as understood by those skilled in the art and includes all technical equivalents that operate in a similar manner to accomplish a similar purpose. It is also to be understood that the mention of one or more steps of a method does not preclude the presence of additional method steps or intervening method steps between those steps expressly identified. Steps of a method may be performed in a different order than those described herein without departing from the scope of the disclosed technology. Similarly, it is also to be understood that the mention of one or more components in a device or system does not preclude the presence of additional components or intervening components between those components expressly identified.

As discussed herein, vasculature of a "subject" or "patient" may be vasculature of a human or any animal. It should be appreciated that an animal may be a variety of any applicable type, including, but not limited thereto, mammal, veterinarian animal, livestock animal or pet type animal, etc. As an example, the animal may be a laboratory animal specifically selected to have certain characteristics similar to a human (e.g., rat, dog, pig, monkey, or the like). It should be appreciated that the subject may be any applicable human patient, for example.

As discussed herein, "operator" may include a doctor, surgeon, or any other individual or delivery instrumentation associated with delivery of a clot retrieval device to the vasculature of a subject.

As discussed herein, "thrombus" can be understood as a clot in the circulatory system that remains in a site of the vasculature hindering or otherwise obstructing flow in a blood vessel. The terms, "clot", "thrombus", "obstruction", "occlusion", "blockage", and/or the like, can be and are often used interchangeably throughout this disclosure.

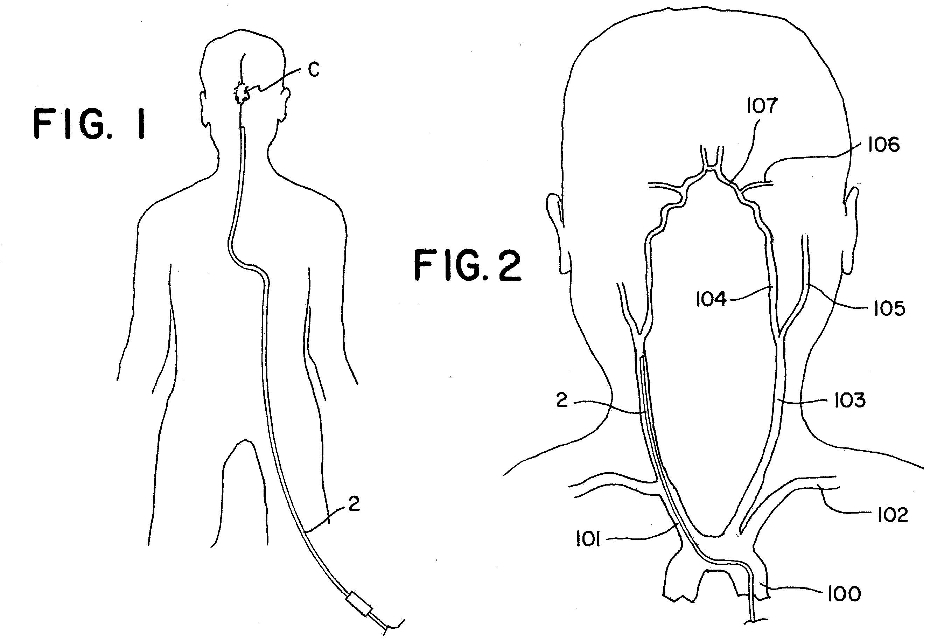

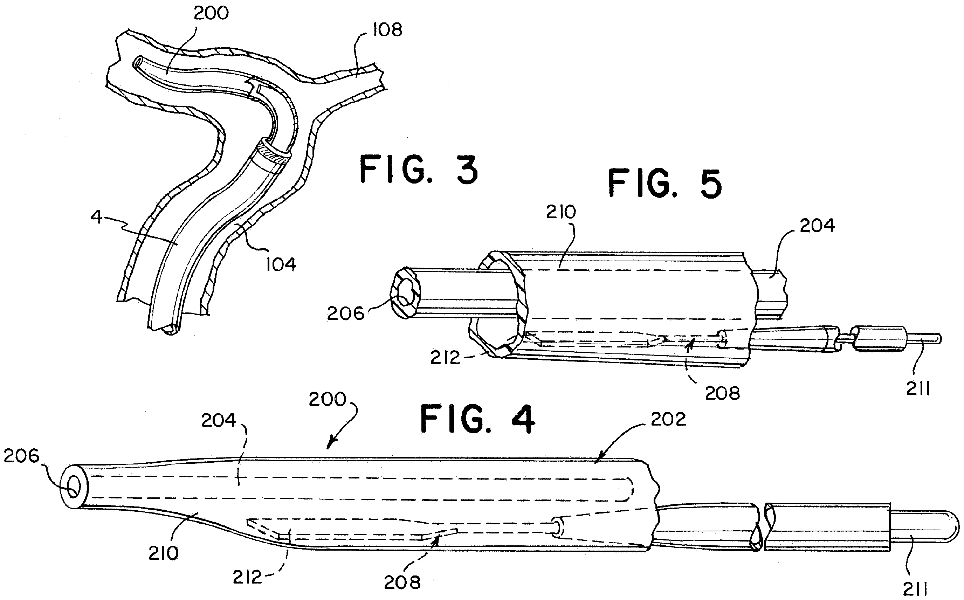

As an example, FIG. 1 depicts a schematic representation of the catheterization of a patient via the femoral artery with a first or base catheter 2 leading to a cerebral clot C. FIG. 2 shows a schematic representation of certain example cerebral vessels. Vessel 100 is the Aorta. Vessel 101 is the brachiocephalic artery. Vessel 102 is the subclavian artery. Vessel 103 is the common carotid artery. Vessel 104 is the internal carotid artery. Vessel 105 is the external carotid artery. Vessel 106 is the middle cerebral artery. Vessel 107 is the anterio-cerebral artery. The catheter 2 from FIG. 1 is shown with its distal end in the common carotid artery. In the more detailed drawings of the invention the details of the access site will not be shown but in general access and delivery is in accordance with FIGs. 1 and 2. FIG. 3 illustrates the distal end of an intermediate or aspiration catheter 4, which can be delivered through the first catheter 2 into the internal carotid artery 104. One critical junction is the ophthalmic ostium 108 and an operator typically needs to avoid that branch to place the catheter into the M1 section of the middle cerebral artery 106.

The present invention discloses examples to aid the operator in traversing the cerebral vessels. One example of an access device 200 is a shapeable and tapered dilator 202 as illustrated in FIGs. 4-6. Dilator 202 can include a base tube 204 having an inner lumen 206. Shapeable element 208 can disposed to one side of the base tube 204 and both of which can be covered by an outer jacket 210. FIG. 3 illustrates that the access device 202 can be directed through the intermediate catheter 4 and is operable to maneuver through the tortious vasculature.

The base tube 204 can be a catheter with a lumen 206 therethrough. The lumen 206 can be used to track along a guidewire (not illustrated) both when both inside and outside the intermediate catheter's 4 lumen. The base tube lumen 206 can be optionally used with guidewires of 0.014, 0.016, 0.018 inches or even larger in diameter. The base tube 204 can be soft and have a low modulus of elasticity, so this material can be rubber-like. This allows the base tube 204 to deform quickly and easily but allows it to recover its shape just as fast. In other examples, the base tube 204 does not have a lumen 206 and travels through the internal carotid artery and to the M1 section of the middle cerebral artery 106 to get to the clot C unguided by a wire.

The shapeable element 208 starts proximally as wire 211 and as it reaches the distal end of the dilator 202, it has a flattened distal end 212. The shapeable element 208 does not extend to the most distal end of the base tube 204, instead being positioned proximal the most distal end. The shapeable element 208 can be made from shape memory or deformable material. This allows the operator to change the shape of the element 208 so it can "steer" the access device 200 inside the vessel.

The material can be, for example, stainless steel so the shapable element 208 can be shaped by the operator. In another example, the shapable element 208 can be Nitinol if the element is to be pre-shaped. The superelastic material memory alloy (Nitinol), a biocompatible alloy of similar properties, or the stainless steel can be in many forms such as wire, strip, sheet, tube or a combination of those shapes. The shapable element 208, in some examples, is not radiopaque, but can be rendered visible under fluoroscopy through the addition of alloying elements (e.g., Platinum, tantalum, etc.) or through a variety of other coatings or marker bands. In addition, alloys that alter shape due to heat or electrical potential can be used.

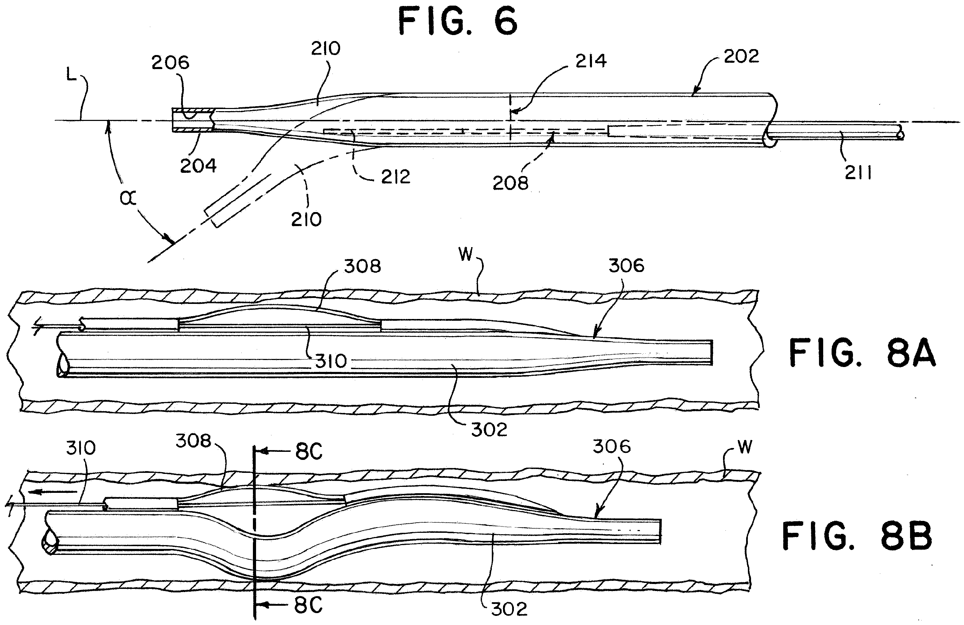

The outer jacket 210 joins the base tube 204 and the shapeable element 208 together and can be a lamination over both. This allows the base tube 204 and the shapeable element 208 to act as one, so when the shapeable element 208 is deformed, the base tube 204 is deformed, and the distal end of dilator 202 is deflected off an axis line L. That deflection can be measured, in some examples, by an angle α from the axis line L (Fig. 6). Separate, or similar to the outer jacket 210, the proximal end 211 of the shapeable element 208 can be optionally coated with heat shrink PET (polyethylene terephthalate) or PTFE (Polytetrafluoroethylene, a.k.a. Teflon^{®}), or directly coated with a low friction material, or could be uncoated.

Any combination of the base tube 204, the shapeable element 208, and outer jacket 210 can be made radiopaque so the dilator 202 can be discernable under fluoroscope. Further, the dilator 202 can have a varying stiffness along its length. For example, transition point 214 can mark where the dilator 202 transitions from a stiffer proximal end to a very soft and flexible distal end. The stiffness transitions can be a factor of any or all three of the components 204, 208, 210 and can be gradually introduced or have a distinct "hinge" point for the transition.

In one example, all three of the components 204, 208, 210 of the dilator 202 are full length, that is to say run from the most proximal position near the operator, to the most distal end as it is exposed in front of the intermediate catheter 4. In a separate example, only the proximal wire end 211 of the shapeable element 208 extends all the way proximally to the operator. The base tube 204, the flattened end 212 and the outer jacket 210 only make up a fraction of the length of the dilator 202. This fraction can be anywhere from 5%-25% of the total length of the dilator 202. This allows for the steerable tip but also reduces delivery time and difficulty. The first and intermediary catheters 2, 4 can be transitioned toward the thrombus C and as the vasculature becomes smaller and/or more tortious, the dilator 202 can be readily introduced to guide the intermediate or microcatheter in or through the internal carotid artery and to the M1 section of the middle cerebral artery 106 to get to the clot C.

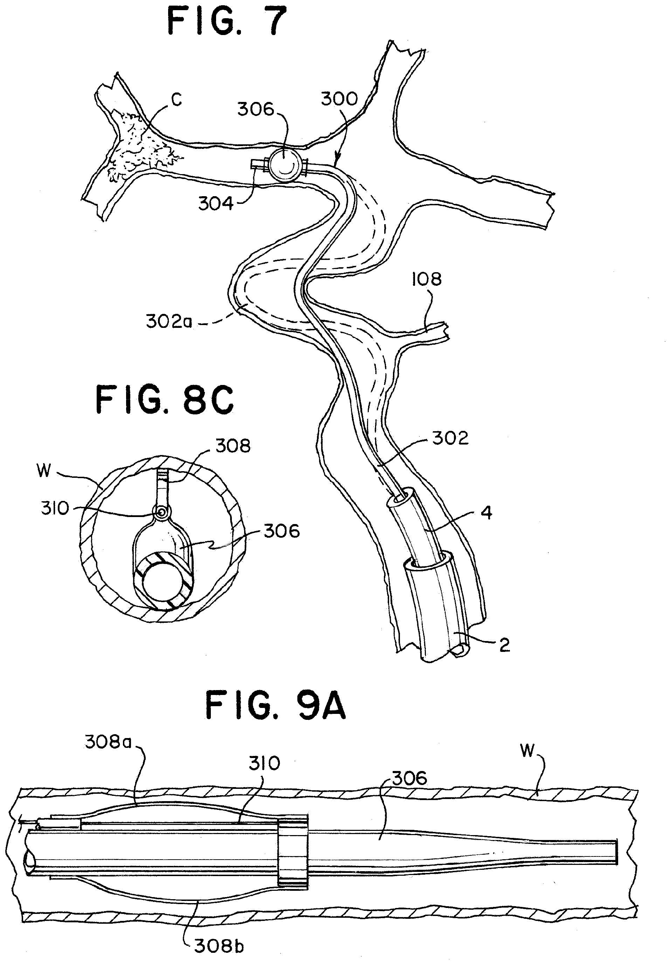

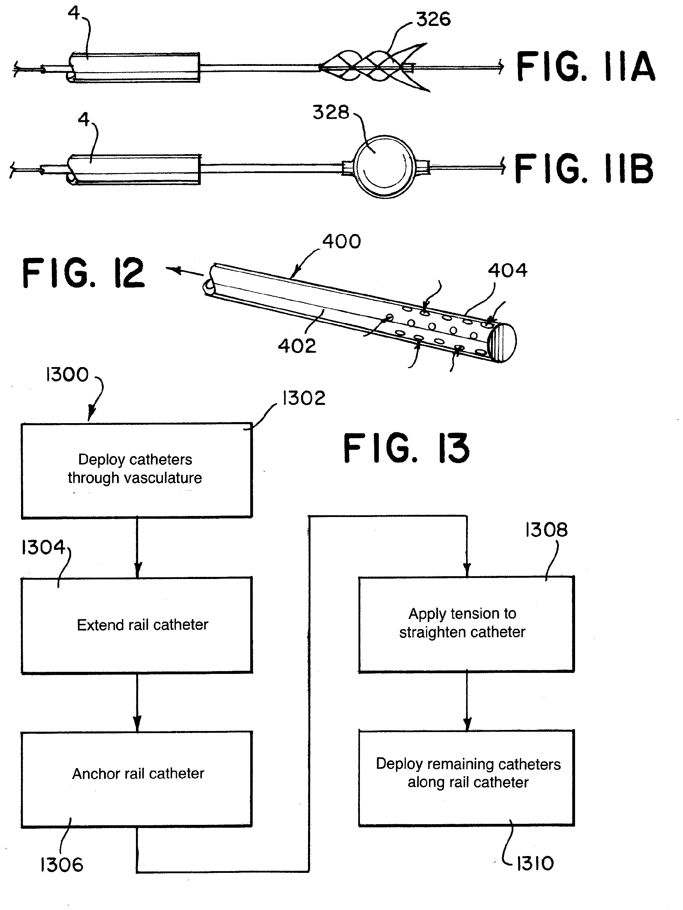

Another example of an access device 300 is a lock and rail catheter 302 as illustrated in FIG. 7. In this example the distal end 304 of the rail catheter 302 is an engaging element 306 that can engage the vessel wall and can anchor the distal end when tension is applied to the catheter 302. In use, as illustrated in FIG. 13, the operator brings up all three catheters 2, 4, 302 and as the catheters are limited based on size, the next smaller size is continued to be advanced. In examples, the base catheter 2 is unlikely to be advanced past the skull base, which is well before the ophthalmic ostium 108, and then the intermediate catheter 4 is typically halted before the ophthalmic ostium 108 or wherever the base and aspiration catheters 2, 4 get stuck on the vasculature (Step 1302). The operator then extends the rail catheter 302 to a point distal to the challenging portion of the anatomy (Step 1304) and anchors the engaging element 306 to the vessel wall (Step 1306). Once anchored, the operator can apply tension to the rail catheter 302 (Step 1308) causing both the catheter and the vasculature to straighten. This straightening draws the rail catheter 302 away from the ophthalmic ostium 108. Now the operator can advance one or both of the base and aspiration catheters 2, 4 along the rail catheter 304 (Step 1310). Since the rail catheter 302 is under straightening tension, the remaining catheters 2, 4 can avoid getting stuck on the ophthalmic ostium 108. As illustrated in FIG. 7 the dashed rail catheter 302a traces the path without being under tension. As can be seen, the dashed path curves close to the ophthalmic ostium 108. Once under tension, the solid line rail catheter 302 straightens the line between the curves in the vessels and avoids being stuck on a junction.

FIGs. 8-12 illustrate different examples of the engaging element 306. FIGs. 8A 8B, and 8C illustrate a flexible anchor 308 that rides flush with the rail catheter 302 during delivery. A pull wire 310 is fixed on the distal end of (FIG. 8A) or distal of (FIG. 8B) the anchor 308. Once the catheter 302 is in place, the operator can tension the pull wire 310 and pull in or shorten the distal end of the anchor 308 or the catheter 302 which can cause the anchor 308 to extend from the side of the catheter 302 and engage the vessel wall W. The engagement can remain as long as the pull wire 310 remains under tension. Once released, the anchor 308/catheter 302 can return to its normal shape and release the vessel wall W and be retrieved. Here, the anchor 308 can be cut from the rail catheter 302 itself. FIG. 8C shows a cross-section of the catheter 302, along the line indicated in FIG. 8B, illustrating the placement of the anchor 308, pull wire 310, and engaging element 306 in relation to the vessel wall W.

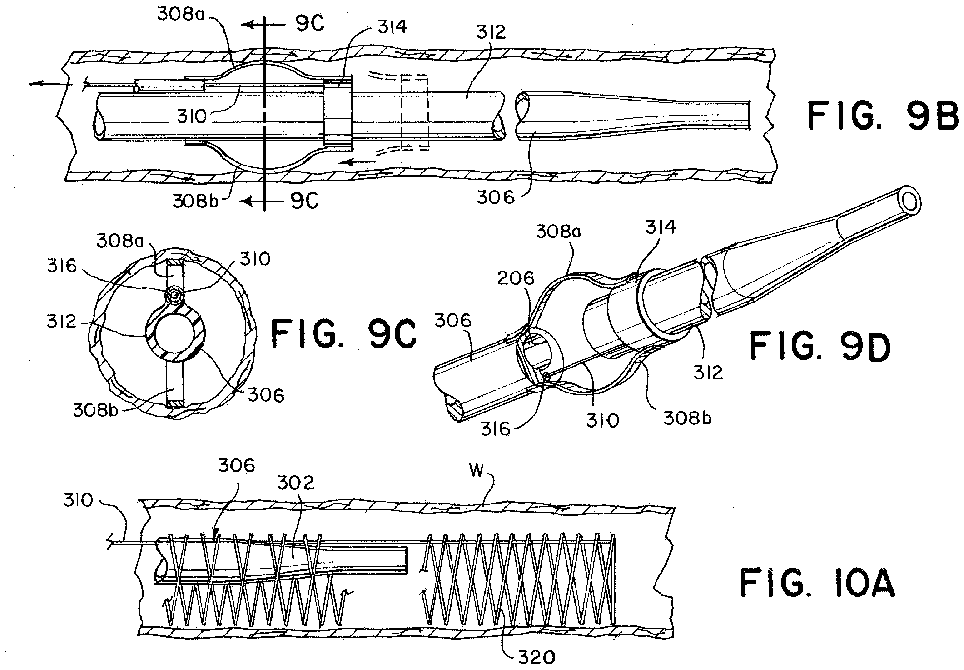

FIGs. 9A, 9B, 9C, and 9D illustrate a double anchor 308a, 308b example of the engaging element 306. FIG. 9A illustrates the delivery position with the anchors 308a, 308b riding approximately flush with the catheter's 302 outer wall 312. Here, like the examples in FIGs. 8A and 8B, either the anchors 308a, 308b or the catheter 302 can be shortened to extend the anchors 308a, 308b to engage the vessel wall W. FIGs. 9B, 9C and 9D illustrate the anchors 308a, 308b and the pull wire 310 connected to a hub 314. The hub 314 can take the form of a ring 314a, 314b (FIGs. 9B, 9C and 9D) that rides over the catheter 302. When the pull wire 310 is tensioned, the ring 314a slides down the catheter 302 and causes the anchors 308a, 308b to expand. The pull wire 310 can have its own lumen 316 external to the outer wall 312 (FIG. 9B and 9D) or a separate lumen 316 inside the outer wall 312, but separate from the guidewire lumen 206. FIG. 9C shows a cross-section of the catheter 302, along the line indicated in FIG. 9B, illustrating the placement of the double anchors 308a and 308b, pull wire 310 with its own lumen 316 external to the outer catheter wall 312, and engaging element 306 in relation to the vessel wall W.

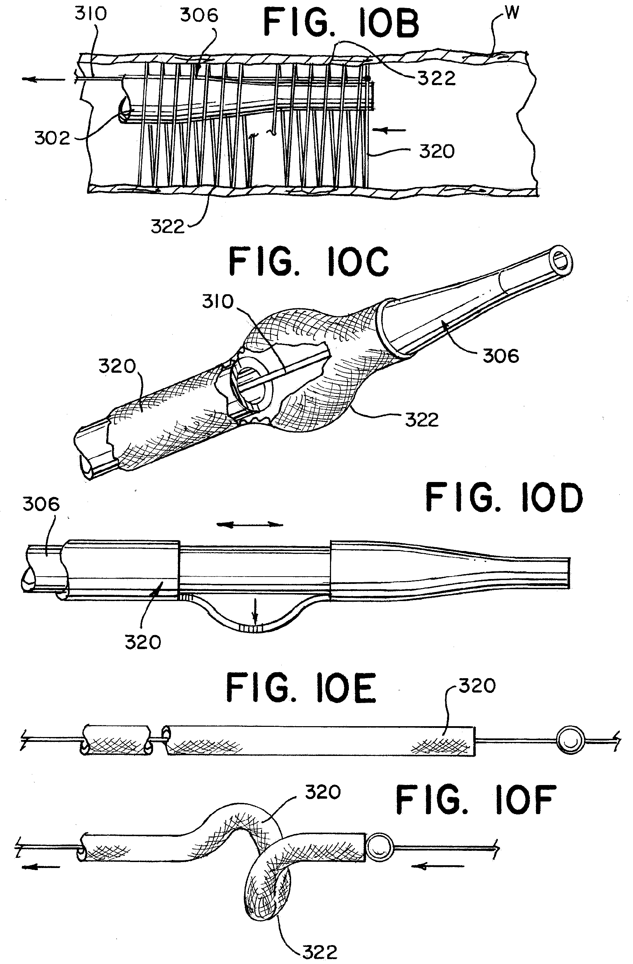

FIGs. 10A, 10B, 10C, 10D, 10E, and 10F illustrate engaging elements 306 where a second catheter or mesh 320 is riding over the rail catheter 302. When the second catheter/mesh 320 is shortened in relation to the rail catheter 302, the second catheter or mesh 320 expands to form an anchor surface 322. FIG. 10A illustrates a second spring cut tube 320 riding over the rail catheter 302. When the pull wire 310 is tensioned, it places the spring 320 in compression and expands to form the anchor surface 322. FIG. 10B is a variant example using a mesh 320, here the mesh can be put into compression from the proximal end, or the pull wire 310 can be tensioned to compress the mesh 320. Here, the mesh anchor surface 322 can be expansive and contact a significant portion of the vessel wall W over a majority of its circumference. FIG. 10C is a vented cut outer catheter 320 that forms anchor surfaces when put into compression. Either a proximal compressive force or the distal portion of the outer catheter is pulled from a tension pull wire 310. FIG. 10D illustrates that the entire distal end of the outer catheter 320 can be "crumpled" into forming an anchor surface 322. FIGs. 10E and 10F illustrate that the entire distal end of the catheter can be curved or twisted into forming an anchor surface 322.

FIGs. 11A and 11B illustrate a different anchoring approach. Here either a stent-like anchor 326 or balloon 328 is advanced ahead of the base and aspiration catheters 2, 4 and used to form an anchor point and rail. These examples may not have a lumen and can have an even smaller delivery profile. The slimmer profile of these rails can allow for a further access up the M1 section of the middle cerebral artery 106.

FIG. 12 illustrates a different example of an access device 400. In this example the catheter 402 can be designed with a porous distal end 404. A vacuum can be applied to the catheter 402 and the porous distal end 404 transfers that suction force to the vessel wall W, causing the distal end to grip the wall with suction.

Access device 200, 300, 400 can be designed for use in the anterior and posterior neurovasculature in vessels such as the internal carotid artery, the M1 and M2 segments of the middle cerebral artery, the vertebral artery, and the basilar arteries. Access device 200, 300, 400 can be delivered endovascularly under fluoroscopic guidance in a similar manner to that of other catheter systems.

The disclosure is not limited to the examples described, which can be varied in construction and detail. The terms "distal" and "proximal" are used throughout the preceding description and are meant to refer to a positions and directions relative to a treating physician. As such, "distal" or distally" refer to a position distant to or a direction away from the physician. Similarly, "proximal" or "proximally" refer to a position near to or a direction towards the physician.

In describing examples, terminology is resorted to for the sake of clarity. It is intended that each term contemplates its broadest meaning as understood by those skilled in the art and includes all technical equivalents that operate in a similar manner to accomplish a similar purpose. It is also to be understood that the mention of one or more steps of a method does not preclude the presence of additional method steps or intervening method steps between those steps expressly identified. Steps of a method can be performed in a different order than those described herein without departing from the scope of the disclosed technology. Similarly, it is also to be understood that the mention of one or more components in a device or system does not preclude the presence of additional components or intervening components between those components expressly identified.

As discussed herein, a "patient" or "subject" can be a human or any animal. It should be appreciated that an animal can be a variety of any applicable type, including, but not limited to, mammal, veterinarian animal, livestock animal or pet-type animal, etc. As an example, the animal can be a laboratory animal specifically selected to have certain characteristics similar to a human (e.g., rat, dog, pig, monkey, or the like).

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 71% to 99%.

By "comprising" or "containing" or "including" or "having" is meant that at least the named compound, element, particle, or method step is present in the composition or article or method, but does not exclude the presence of other compounds, materials, particles, method steps, even if the other such compounds, material, particles, method steps have the same function as what is named.

It must also be noted that, as used in the specification and the appended claims, the singular forms "a," "an," and "the" include plural referents unless the context clearly dictates otherwise. Ranges can be expressed herein as from "about" or "approximately" one particular value and/or to "about" or "approximately" another particular value. When such a range is expressed, other exemplary embodiments include from the one particular value and/or to the other particular value.

The descriptions contained herein are examples of the disclosure and are not intended in any way to limit the scope of the disclosure. While particular examples of the present disclosure are described, various modifications to devices and methods can be made without departing from the scope and spirit of the disclosure. For example, while the examples described herein refer to particular components, the disclosure includes other examples utilizing various combinations of components to achieve a described functionality, utilizing alternative materials to achieve a described functionality, combining components from the various examples, combining components from the various example with known components, etc. The disclosure contemplates substitutions of component parts illustrated herein with other well-known and commercially-available products. To those having ordinary skill in the art to which this disclosure relates, these modifications are often apparent and are intended to be within the scope of the claims which follow.

## Claims

1. A cerebral vessel access device comprising:
a base tube comprising an inner lumen;
a shapeable element disposed to one side of the base tube; and
an outer jacket covering the base tube and a portion of the shapable element,
wherein the shapable element is configured to deflect from an axis line causing the base tube and outer jacket to deflect along with the shapable element.

2. The cerebral vessel access device of claim 1, wherein the shapeable element comprises:
a flattened end located near the base tube and covered by the outer jacket, and
a proximal wire connected to the flattened end and a majority of the wire is disposed outside the outer jacket.

3. A method of cerebral vessel access, comprising the steps of:
deploying at least a base catheter and a rail catheter into the cerebral vessels (Step 400);
extending the rail catheter to an obstruction in the cerebral vessel (Step 402);
anchoring an engaging element to a wall of the cerebral vessel (Step 404);
applying tension to the rail catheter, partially straightening at least the rail catheter (Step 406); and
deploying at least the base catheter along the tensioned rail catheter (Step 408).
